# EUROPEAN PATENT APPLICATION

(11) **EP 2 574 353 A1**
(43) Date of publication of application: **03.04.2013**
(21) Application number: 12181511.2
(22) Date of filing: 23.08.2012
(51) Int. Cl.: A61M 1/36

(54) **Device to encourage blood circulation through a vascular access device during periods between dialysis treatments**

(30) Priority: 30.09.2011 US 201113249366
(71) Applicant: Covidien LP, Mansfield, MA 02048 (US)
(72) Inventor: Sansoucy, Michael, Wrentham, Massachusetts 02093 (US); Bellisario, Marc, Tewksbury, Massachusetts 01876 (US)
(74) Representative: Gray, James

(57) **Abstract**

A vascular access system includes a vascular access device and a portable recirculation device (10). The vascular access device defines at least one lumen and is configured and dimensioned to be positioned within a blood vessel of a patient. The recirculation device includes a housing (12) defining a channel (14) having an inlet port (16a) and an outlet port (16b) for passage of blood through the channel. The channel includes a pump (18) for circulating blood through the vascular access device.

## Description

### CROSS REFERENCE TO RELATED APPLICATION

This application claims the benefit of and priority to U.S. Application Serial No. 13/249,366 which was filed on September 30, 2011.

### TECHNICAL FIELD

The present disclosure relates to vascular access devices. More particularly, the present disclosure relates to vascular access systems including a recirculation device for circulating blood through a vascular access device between dialysis treatments.

### DESCRIPTION OF RELATED ART

Dialysis or hemodialysis is a procedure used to provide an artificial replacement for lost or reduced kidney function in people with renal failure. Hemodialysis may be used for those with acute disturbance in kidney function as well as those with chronic kidney disease. Those with chronic kidney disease or chronic renal failure require hemodialysis at regular intervals until a renal transplant can be performed.

For a patient suffering from lost or reduced kidney function, a hemodialysis procedure is required about three times per week and each procedure takes about 3-5 hours to perform. During a hemodialysis procedure, a patient's blood is withdrawn from the patient through a vascular access device, such as a catheter, and is pumped through a dialyzer to expose the blood to a partially permeable membrane formed of synthetic hollow fibers. The blood flows through the fibers as a dialysis solution flows around the outside of the fibers such that water and waste are removed from the blood. The cleansed blood is then returned to the patient through the vascular access device. The patient's blood may be accessed through a native vein, formed fistula, an artificial vessel or vascular graft, or a catheter.

Complications may arise from the use of vascular access devices, with the risk of complications increasing with increased duration of implantation. Common complications include venous stenosis, fibrin sheath, thrombosis, infection, and occlusion of the vascular access device. For example, a catheter can become occluded by a thrombus. In order to prevent clotting of catheters in blood vessels between uses, such as, for example, between dialysis treatments when the catheter is essentially sitting inside a vein without flow, the lumens of the catheter are often filled with a lock solution that includes a concentrated solution of heparin, a commonly used anticoagulant. In this configuration, however, stagnant blood at the tip of the catheter can cause thrombus and flow problems within the device. Additionally, artificial vessels and vascular grafts may be formed from materials, such as polytetrafluoroethylene, which encourage growth of endothelial cells that could lead to thrombus during periods of low or no flow through the vascular access device, such as between dialysis treatments.

Ensuring a stable and adequate amount of blood flow through a vascular access device between dialysis sessions would lead to an improved dialysis effect, decreased risk of complications, such as thrombus formation, and extended service life of the vascular access device.

### SUMMARY

A vascular access system in accordance with the present disclosure includes a vascular access device and a portable recirculation device. The vascular access device defines at least one lumen and is configured and dimensioned to be positioned within a blood vessel of a patient. The portable recirculation device includes a housing defining a channel having an inlet port and an outlet port for passage of blood through the channel. The channel includes a pump for circulating blood through the at least one lumen of the vascular access device.

The vascular access device may be, for example, a catheter, a port access device, a shunt, an arteriovenous fistula or graft, or an arterial graft or venous graft. In embodiments, the vascular access device is a catheter. In embodiments, the vascular access device is a graft.

The recirculation device may be integrally formed with, or releasably attachable to, the vascular access device. In embodiments, the recirculation device may include at least one adapter adapted to be releasably attached to the vascular access device. In embodiments, access needles may be utilized to connect the vascular access device to the recirculation device.

The recirculation device may be dimensioned to be worn on a body of a patient or may be implantable.

The pump of the recirculation device may be a fluid displacement pump. In embodiments, the pump may include a motor powered by a battery for rotating an impeller within the channel of the housing. In embodiments, the pump is a peristaltic pump.

The recirculation device may include at least one sensor disposed within the housing. The sensor may be electrically connected to a transmitter for transmitting data to an indicator provided on an outer surface of the housing. In embodiments, the sensor measures solute concentration in blood. In embodiments, the sensor is a pressure sensor operably connected to the pump.

The recirculation device may include valves for controlling the flow of fluid. The valves may be open to allow fluid flow through a dialysis circuit or may be closed to block fluid flow. In embodiments, the valves may include a side port to allow for the introduction of agents into the recirculation device.

### BRIEF DESCRIPTION OF DRAWINGS

FIG. 1A is a perspective view of a recirculation device in accordance with an embodiment of the present disclosure;
FIG. 1B is a perspective view within the housing of the recirculation device of FIG. 1A;
FIG. 2 is a top view of a vascular access system including a catheter and a recirculation device in accordance with an embodiment of the present disclosure;
FIG. 3 is a perspective view of a recirculation device in accordance with another embodiment of the present disclosure;
FIG. 4 is a schematic illustration of a vascular access system including a graft and a recirculation device in accordance with an embodiment of the present disclosure; and
FIG. 5 is a schematic illustration of a vascular access system including a graft and a recirculation device in accordance with another embodiment of the present disclosure.

### DETAILED DESCRIPTION

Various exemplary embodiments of the present disclosure are discussed hereinbelow in terms of a vascular access system including a vascular access device and a recirculation device that is integrally or releasably attached to the vascular access device to provide blood flow therethrough during periods in which the fluid flow rate through the vascular access device is minimal or non-existent, e.g., between dialysis treatments. The recirculation device is entirely portable such that a patient is completely ambulatory during use. The vascular access device may be any device that can be used for vascular access, such as temporary or permanent indwelling catheters, port access devices, shunts, arteriovenous fistulas and grafts, and/or arterial grafts or venous grafts.

In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse, or other care provider and may include support personnel.

The following discussion includes a description of embodiments of the presently disclosed vascular access system that includes a recirculation device that provides blood flow rates capable of minimizing thrombus within a vascular access device in accordance with the principles of the present disclosure.

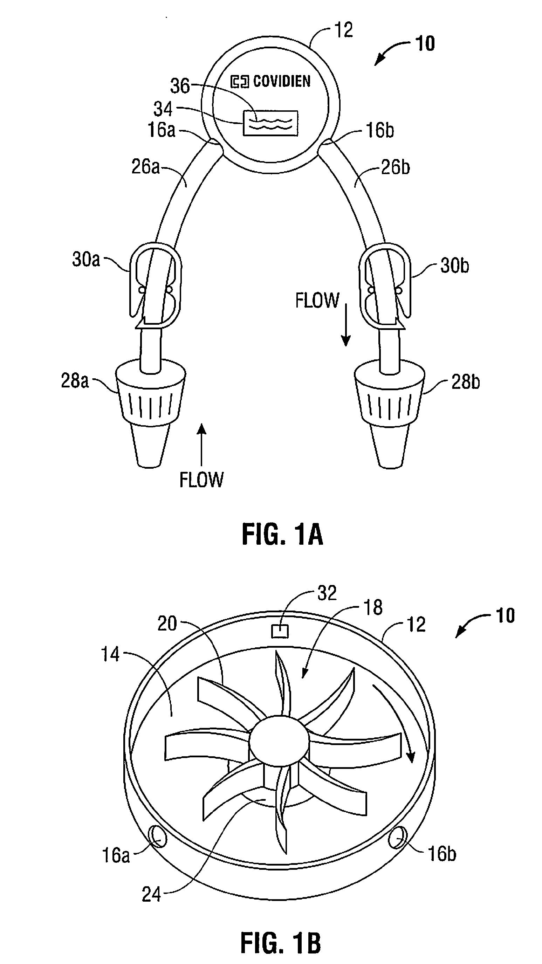

Referring now to the figures, wherein like components are designated by like reference numerals throughout the several views, FIGS. 1A and 1B illustrate one embodiment of a recirculation device 10 for use with a vascular access system of the present disclosure for circulating blood within a vascular access device (not shown). The recirculation device 10 includes a housing 12 defining a channel 14 for passage of blood therethrough via inlet and outlet ports 16a, 16b. The channel 14 includes a pump 18 for moving fluids, i.e., blood. The pump 18 may be a gear pump, a screw pump, a lobe pump, a peristaltic pump, a plunger pump, a diaphragm pump, a pulsatile pump, a centrifugal pump, among other fluid displacement pumps within the purview of those skilled in the art. It should be understood that the pump may be chosen based on the shear stress exerted on the blood by the pump, for example where lower shear stress is desirable. Alternately, or in addition, the pump may be chosen based on the energy requirements for operating the pump, or other desirable pump characteristics.

As illustrated in the present embodiment, the pump 18 may include an impeller 20 to control the flow rate of blood therethrough. The impeller 20 rotates via energy supplied by a battery 22 (not shown) to a motor 24 that drives the impeller 20. The impeller 20 rotates blood outwardly from the center of rotation thereby creating pressure within the confines of the housing 12.

The impeller 20 and/or other fluid contacting surfaces of the recirculation device 10 are fabricated from a biocompatible material. It is envisioned that the impeller 20 and the housing 12 may be made from any of a variety of polymeric and/or metallic materials. The impeller 20 and the housing 12 may be coated with one or more therapeutic agents, such as anti-coagulants, anti-infectives, anti-microbials, antibacterials, anti-proliferatives, anti-inflammatories, anti-adhesives, antibiotics, thrombolytic agents, and other agents that have clinical use. Specific agents within these classes are within the purview of those skilled in the art and are dependent upon such factors as, for example, the type of vascular access device in which the therapeutic agent is utilized and the duration of use (e.g., a polymeric heparin-containing coating).

The battery 22 may be one or more internal or external power cells, such as, for example, a nickel cadmium type battery, an alkaline battery, or a lithium battery. In embodiments, the battery 22 may disengage from the recirculation device 10 for recharging and/or replacing the battery 22. In other embodiments, the battery 22 may be rechargeable from within the recirculation device. For example, recirculation device 10 may include a magnetically suspended impeller 20 connected to a drive mechanism having an inductance rechargeable battery 22.

The battery 22 powers a drive mechanism (not shown) within the motor 24 to control the frequency of rotation of the impeller 20 and thus, the flow rate of blood through the housing 12. The rotational speed of the impeller 20 should be controlled such as not to impart shear stress to blood or create high pressures, e.g., greater than about - 250 mmHg, which can damage or lyse blood cells. It should be understood by those skilled in the art that the rotational speed of the impeller 20 should be tailored to the blood vessel to which it is attached. For example, the rate of blood flow through superior vena cava is about 1800 mL/min while the rate of blood flow in the vasculature of the forearm is less than that of the superior vena cava. Accordingly, the rotational speed of the impeller 20 as well as the size, shape, and cross-sectional area of the impeller 20 and/or channel 14 of the housing 12 should be dimensioned to provide the appropriate flow rate to blood moving therethrough and/or be capable of operating at various speeds.

The housing 12 is adapted to fluidly couple to a vascular access device (not shown). In some embodiments, the recirculation device 10 may be implanted within a patient, e.g., subcutaneously. The housing 12 may include extension tubes 26a, 26b including adapters 28a, 28b integrally formed, or attached thereto, extending from the housing 12 for attachment to a vascular access device. Clamps 30a, 30b may be positioned on the extension tubes 26a, 26b respectively, to control the flow of fluid therethrough. In embodiments, the ports 16a, 16b of recirculation device 10 may be adapted to directly engage and fluidly couple a vascular access device, such as the luer adapters of a catheter as described in detail below.

The recirculation device 10 may include one or more integrated sensors 32 for sensing properties of, or changes to, the blood passing therethrough. The sensors 32 may monitor parameters related and unrelated to dialysis, such as blood pressure, solute levels, thrombus formation, oxygen saturation, among other parameters relevant to patient health. In embodiments, the sensor 32 may be used to identify solutes in the blood, such as glucose, sodium, potassium, and urea.

The sensor 32 may be disposed within the housing 12 to facilitate blood flow across or over the sensor 32. In embodiments, the sensor 32 is electrically connected to a transmitter 34 for transmitting data to an indicator 36 (FIG. 1A) which may provide a visual indication on an outer surface of the housing 12 and/or an audible signal that identifies the presence and/or amount of a particular preselected parameter measured by the sensor 32. In embodiments, this visual indication or audible signal may indicate to the patient that dialysis is needed. Alternatively, the transmitter 34 may store data and later transmit the data to an external receiving unit (not shown) for analysis by a clinician. The sensor 32 may take continuous or intermittent measurements. In some embodiments, the sensor 32 may trigger the functioning of the pump 18. For example, a pressure sensor for measuring the blood flow through the recirculation device 10 may be operably connected to the pump 18 to adjust the rotational speed of the impeller 20 to maintain an adequate flow rate through the vascular access device.

The sensor 32 may be an image sensor such as a CCD or CMOS image sensor; a sound sensor such as ultrasound; a light sensor such as a photodiode; or other electrical or electrochemical sensor for measuring characteristics such as resistivity, impedance, temperature, pH, enzymatic activity, etc. of blood. Other suitable sensors 32 include, for example, microoptical detectors for detection of particle size, electrochemical detectors, acoustic, or electrical sensors to detect blood content or other sensors that would be sensitive to characteristics of the blood flowing there past.

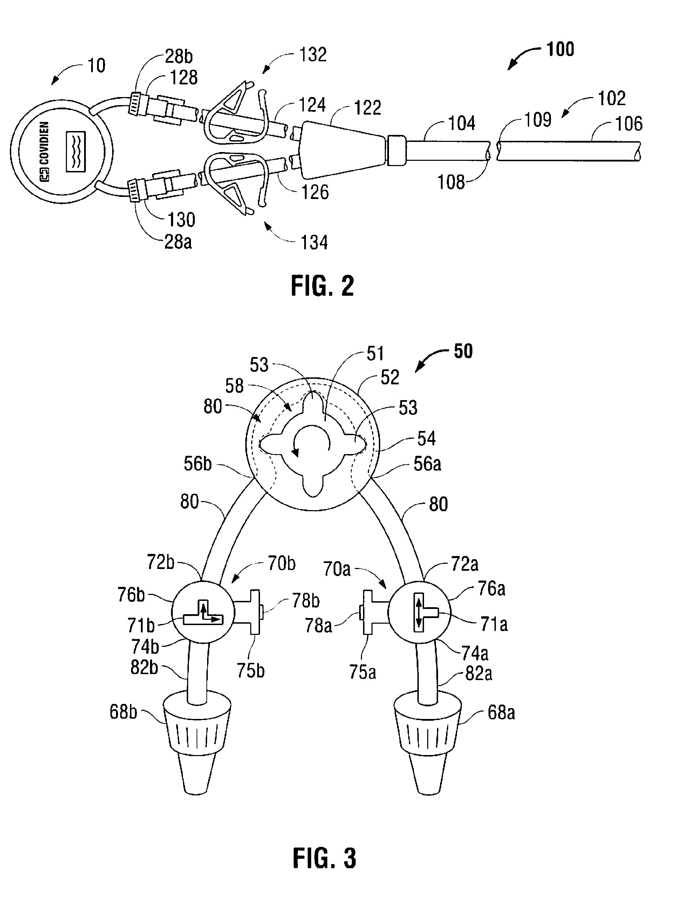

The recirculation device 10 may be utilized with a variety of vascular access devices. A catheter 100 for use with the recirculation device 10 is illustrated in FIG. 2. While a dual lumen catheter is described below, it should be appreciated that the principles of the present disclosure are equally applicable to catheters having any number of lumens, such as triple lumen catheters, and other catheters of various cross-sectional geometries, tip configurations, and/or catheters that are employable in a variety of other medical procedures. Suitable non-exclusive examples of catheters falling within the scope of the present disclosure include, for example, the PALINDROME™ and MAHURKAR^{®} Maxid™ catheters, each of which is made available by Covidien, which maintains a principal place of business at 15 Hampshire Street, Mansfield, Massachusetts.

The catheter 100 may include an elongate body 102, a catheter hub 122, and extension tubes 124, 126. The elongate body 102 includes a proximal end portion 104 and a distal end portion 106, and defines lumens 108, 109 through which blood or other fluids may be removed and/or returned from or to a patient. In the depicted embodiment, the elongate body 102 has a cylindrical shape. Alternatively, the elongate body 102 may have any suitable shape or configuration. The lumens 108, 109 of the elongate body 102 are adapted to be fluidly coupled to the catheter hub 122. The extension tubes 124, 126 extend proximally from the catheter hub 122 and may include adapters 128, 130, respectively, attached thereto for attachment to external devices. Clamps 132, 134 may also be positioned on the extension tubes 124, 126, respectively, to control the flow of fluid through extension tubes 124, 126 by inhibiting or permitting the passage of fluid upon clamping or unclamping.

In use during a dialysis treatment session, the adapters 128, 130 are connected to an external device (not shown), such as a hemodialysis unit, so that blood may be removed from the patient, cleansed by the hemodialysis unit, and delivered back to the patient. After use, the adapters 128, 130 can be disconnected from the external device and releasably coupled to the adapters 28a, 28b of the recirculation device 10. Mechanisms for selective coupling and decoupling of the recirculation device 10 with the catheter 100 include male/female fasteners, threaded connections, snap fittings, friction fittings, tongue and groove arrangements, cam-lock mechanisms, among other mating structures that provide a releasable fluid tight seal between the recirculation device 10 and the catheter 100. The housing 12 of the recirculation device 10 may be carried or worn by a patient. The recirculation device 10 provides constant blood flow through the catheter 100 at adequate flow rates to prevent complications produced by stagnant blood, such as thrombus.

FIG. 3 illustrates another embodiment of a recirculation device 50 in accordance with the present disclosure. The recirculation device 50 is a peristaltic pump including a housing 52 defining a channel 54 including a pump 58 and flexible tubing 80. The tubing 80 extends through inlet and outlet ports 56a, 56b of the housing 52 for attachment to a vascular access device (not shown). Pump 58 includes a rotating pump head 51 having a plurality of rollers 53 extending radially therefrom for engagement with the tubing 80. The tubing 80 is pinched, squeezed, pressed, or otherwise impinged by the rollers 53 within the confines of the channel 54 such that the alternation between compression of the tubing 80 and release of the tubing 80 generates suction and discharge pressure to move fluid therethrough. As understood by those skilled in the art, the flow rate through the tubing may be influenced by tubing diameter, pump head configuration, among other factors within the purview of those skilled in the art.

Valves 70a, 70b may be positioned on the tubing 80 to control the flow of fluid therethrough. Valves 70a, 70b may be integrally formed with tubing 80. Alternatively, the tubing 80 may be fluidly coupled with first openings 72a, 72b of valves 70a, 70b, respectively, and extension tubes 82a, 82b may be fluidly coupled with second openings 74a, 74b of valves 70a, 70b, respectively. In embodiments, adapters 68a, 68b may be integrally formed with, or attached to, the extension tubes 82a, 82b for attachment to a vascular access device. In some embodiments, the valves 70a, 70b may be adapted to directly engage and fluidly communicate with a vascular access device.

Valves 70a, 70b may be stop cock valves which include a main body 76a, 76b having a first opening 72a, 72b, a second opening 74a, 74b, and optionally one or more side ports 78a, 78b. A rotary valve 71a, 71b is disposed within the main body 76a, 76b and is connected to an external handle 75a, 75b such that rotation of the handle 75a, 75b allows communication or blocking between the first opening 72a, 72b, the second opening 74a, 74b, and/or the side port 78a, 78b of the valve 70a, 70b. As illustrated, valve 70a is shown in a first position for allowing fluid flow between the first opening 72a and second opening 74a. When valves 70a, 70b are both in the first position, the dialysis circuit is open. Valve 70b is shown in a second position which closes the dialysis circuit and allows for fluid flow between the first opening 72b and the side port 78b. When valves 70a, 70b are in the second position, a limited circuit is formed within the recirculation device 50 and associated tubing 80. In embodiments, the side port 78b may be utilized for cleaning and/or debulking of thrombus (e.g., through the use of lytics) from the recirculation device 50. Other valve configurations are also envisioned, such as a valve having an open side port when the dialysis circuit is open for introduction of therapeutic agents into the blood.

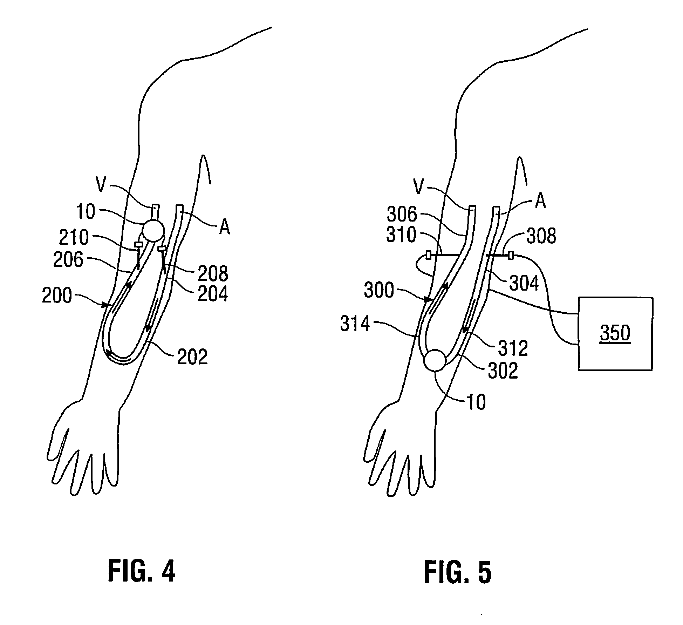

Referring now to FIG. 4, a vascular access system including a graft 200 including a recirculation device 10 is illustrated. While shown and described below as a forearm loop arteriovenous graft, it should be appreciated that the principles of the present disclosure are equally applicable to a variety of graft configurations for placement in a variety of locations within a patient's body.

Graft 200 includes hollow tubular body 202 having an arterial end 204 and a venous end 206 that are anastomosed between an artery "A" and a vein "V", respectively. During a dialysis treatment session, graft 200 is connected to a hemodialysis unit (not shown) by access needles 208, 210 such that blood is withdrawn from the arterial end 204, enters the hemodialysis unit for removal of impurities from the blood, and is returned through the venous end 206. After the dialysis session, recirculation device 10 may be operably connected to the access needles 208, 210 to maintain adequate blood flow through graft 200 until the next dialysis treatment session.

In other embodiments, as illustrated in FIG. 5, graft 300 includes a tubular body 302 having a recirculation device 10 integrally formed with segments 312, 314 of tubular body 302 for subcutaneous implantation. Segment 312 includes an arterial end 304 and segment 314 includes a venous end 306 that are anastomosed between an artery "A" and a vein "V", respectively. Dialysis is performed by connecting the graft 300 to a hemodialysis unit 350 via access needles 308, 310. After dialysis is complete, the access needles 308, 310 may be removed and adequate blood flow through graft 300 may be maintained by recirculation device 10.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the system based on the above-described embodiments. Accordingly, the present disclosure is not to be limited by what has been particularly shown and described, except as indicated by the appended claims.

## Claims

1. A vascular access system comprising:
a vascular access device defining at least one lumen and being configured and dimensioned to be positioned within a blood vessel of a patient; and
a portable recirculation device including a housing defining a channel having an inlet port and an outlet port for passage of blood through the channel, the channel including a pump for circulating blood through the at least one lumen of the vascular access device.

2. The vascular access system of claim 1, wherein the vascular access device is a catheter.

3. The vascular access system of claim 1, wherein the vascular access device is a graft.

4. The vascular access system of any one of the preceding claims, wherein recirculation device includes at least one adapter adapted to be releasably attached to the vascular access device.

5. The vascular access system of any one of claims 1-3, wherein the recirculation device is integrally formed with the vascular access device.

6. The vascular access system of any one of claims 1-3 and 5, wherein the recirculation device is implantable.

7. The vascular access system of any one of the preceding claims, wherein the pump includes a motor powered by a battery for rotating an impeller within the channel of the housing.

8. The vascular access system of any one of the preceding claims, wherein the recirculation device includes at least one sensor disposed within the housing.

9. The vascular access system of any one of claims 1-4, 7 and 8, wherein the sensor is electrically connected to a transmitter for transmitting data to an indicator provided on an outer surface of the housing.

10. The vascular access system of any one of the preceding claims, wherein the sensor measures solute concentration in blood.

11. The vascular access system of any one of claims 1-9, wherein the sensor is a pressure sensor operably connected to the pump.

12. The vascular access system of any one of the preceding claims, wherein the pump is a peristaltic pump.

13. The vascular access system of any one of the preceding claims, wherein the recirculation device further includes valves for controlling the flow of fluid therethrough.

14. The vascular access system of any one of the preceding claims, further comprising access needles for connecting the vascular access device to the recirculation device.
